# EUROPEAN PATENT APPLICATION

(11) **EP 1 306 090 A1**
(43) Date of publication of application: **02.05.2003**
(21) Application number: 01953305.8
(22) Date of filing: 18.07.2001
(51) Int. Cl.: A61K 31/80, A61K 7/00, A61K 7/48, A61P 17/00, A61P 17/16, A61P 43/00

(54) **COSMETICS OR DERMATOLOGICAL PREPARATIONS CONTAINING POLYMERS HAVING PHOSPHORYLCHOLINE SIDE CHAIN**

(30) Priority: 04.08.2000 JP 2000236902
(71) Applicant: SHISEIDO COMPANY LIMITED, Chuo-ku, Tokyo 104-8010 (JP)
(72) Inventor: KATAGIRI, Chika, Yokohama-shi, Kanagawa 236-8643 (JP); NAKANE, Toshihiko, Yokohama-shi, Kanagawa 224-8558 (JP); MARUYAMA, Nao, Yokohama-shi, Kanagawa 224-8558 (JP); HIRAO, Tetsuji, Yokohama-shi, Kanagawa 236-8643 (JP)
(74) Representative: Hiebl, Inge Elisabeth
(86) International application number: JP0106221
(87) International publication number: WO02011738

(57) **Abstract**

Provided is a cosmetic or a dermatological composition comprising as an active ingredient, a specific acryl base copolymer having phosphorylcholine as a side chain or a pendant group. Such active ingredient can inhibit or repair skin damages by strengthening the expression of a β-glucocerebrosidase gene.

## Description

### Technical Field

The present invention relates to a cosmetic or a preparation for inhibiting or repairing dermatological skin damages.

### Background Art

A barrier function of a skin depends on a lamella structure of intercellular lipids in the stratum corneum constituted primarily from ceramide, cholesterol and free fatty acids. This intercellular lipid originates in exocytosis of lamellar bodies. The lamellar body is an intracellular organ containing a lot of lipid present in a spinous stratum and a granular stratum. Ceramide, cholesterol and free fatty acid each constituting a lamellar structure are stored in lamellar bodies in the forms of glucosyl ceramide, cholesterol and phospholipid respectively. β-Glucocerebrosidase (hereinafter referred to as GCase) is an enzyme which cuts sugar of glucosyl ceramide stored in lamellar bodies to convert it into ceramide, and it is an essential enzyme for forming a barrier function. It has been found that in, for example, a Gaucher disease originating in genetical deficiency of GCase, a lamellar structure of intercellular lipid is broken to cause barrier function fault. Further, it is reported that topical application of a GCase inhibitor breaks a lamellar structure and retards recovery of a barrier function.

It is well known that dry environment causes skin troubles such as rough skin, and it is known as well that persons who are intrinsically liable to cause skin troubles are present and that skin troubles are brought about by aging. However, the mechanisms of rough skin caused by drying and barrier function deficiency have not sufficiently be researched, and protecting drugs against skin damages caused particularly by drying paying attentions to metabolic enzymes have not yet been researched as well. In general, substances having a hygroscopicity or a moisturizing property which- can supply moisture to a horny layer have so far been used as treatment against such skin troubles. It is known as well that a large variety of substances having such action is useful for preventing skin troubles.

On the other hand, it is proposed to use a substance for accelerating a ceramide synthetic enzyme (serine-palmitoyl transferase: SPT) activity paying attentions to skin physiology to improve a skin barrier function (Japanese Patent Application Laid-Open No. 194383/1997).

As described above, it is recognized that a specific moisturizing agent is effective for preventing or inhibiting skin troubles or skin barrier function damages caused by, for example, drying. However, still present is demand to substances which can surely inhibit such skin barrier function damages and which can, if necessary, repair or strengthen a reduction in a skin barrier function to a higher level than a function of a normal skin.

It is indicated in Japanese Patent Application Laid-Open No. 194383/1997 described above that an SPT activity-accelerating substance can improve a skin barrier function, but it is not based on direct data regarding under what situation the SPT is damaged. Accordingly, it is not necessarily clear that the SPT activity-accelerating substance is suited to "improving" a reduction in a skin barrier function brought about by what cause.

### Disclosure of the Invention

On the other hand, the present inventors have investigated the relation of the skin with a lipid metabolic enzyme taking part in a skin barrier function in the keratinocytes, and as a result thereof, they have confirmed that the expression of gene coding
β-glucocerebrosidase (GCase) and the activity of GCase is reduced by drying.

Further, the present inventors have found that a specific copolymer (FRAGRANCE JOURNAL, 1998-7, p. 97-) having an ethyl phosphorylcholine part as a pendant group, which is known to have almost the same moisturizing property and hygroscopicity as those of glycerin known as a typical moisturizer not only inhibits more strongly a reduction in the expression of GCase gene in the keratinocytes described above as compared with a conventional moisturizer such as glycerin but also can significantly repair the expression of GCase gene damaged by drying up to the normal level of expression of Gcase in the keratinocytes which is not exposed to such damage. The present invention is based on such knowledge.

Thus, the present invention provides a composition for inhibiting or repairing a reduction in the expression of a GCase gene, in its turn strengthening a skin barrier function, comprising as an active ingredient, a copolymer represented by Formula (I): 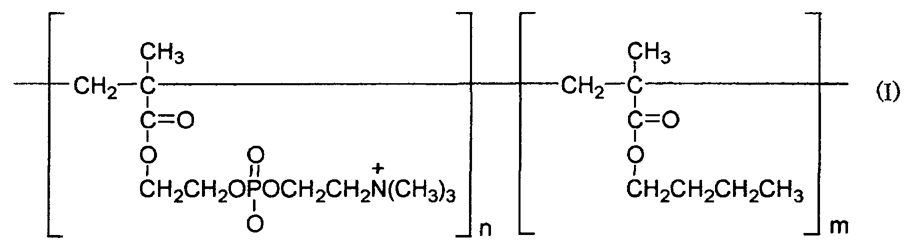 wherein m and n represent independently integers in which the total thereof allows a number average molecular weight of the above copolymer to be 1,000 to 100,000, and a ratio of m to n falls in a range of 19 : 1 to 1 : 2.

Further, provided as well is a method for inhibiting or repairing a reduction in a skin barrier function or strengthening the skin barrier function, comprising percutaneously administrating the copolymer represented by Formula (I) described above to the skin of an individual which is likely to be reduced in expression of GCase gene in the keratinocytes to strengthen the expression of the GCase gene in the epidermal keratinocytes of the skin.

Further, provided as well is use of the copolymer represented by Formula (I) for preparing a composition for strengthening a skin barrier function.

### Brief Description of the Drawings

Fig. 1 is a graph showing a time-dependent change in GCase activity caused by exposure of a cultured human keratinocytes to the air (average ±SE, n = 4 to 8, **: p<0.01, *** p<0.001).

Fig. 2 is a graph showing a time-dependent change in an expression level of GCase gene caused by exposure of cultured human keratinocytes to the air.

Fig. 3 is a graph showing that poly(MPC-co-BMA) enhances dependently on a concentration expression of a GCase gene reduced by exposure of cultured human keratinocytes to the air.

### Best Mode for Carrying out of the Invention

The copolymer represented by Formula (I) described above [hereinafter referred to as poly(MPC-co-BMA)] may take any integer of m and any average value of n and may have any distributions of them as long as it meets the objects of the resent invention in the ranges described above. Usually, however, m and n are independently integers in which the total thereof allows a number average molecular weight of the above copolymer to be 5,000 to 50,000, preferably 5,000 to 10,000, and a ratio of m to n falls in a range of 10 : 1 to 1 : 2, preferably 5 : 1 to 1 : 1 and particularly preferably about 4 : 1. A part of such copolymer is described in FRAGRANCE JOURNAL described above, and it can be produced based on a method described in a literature cited therein.

Poly(MPC-co-BMA) described above not only can inhibit a reduction in a GCase activity or the expression of a GCase gene in human keratinocytes caused by drying but also can repair a keratinocyte damaged by drying up to an expression level of a GCase gene in a human keratinocyte which stays in a normal state.

The actions and effects of the present invention not only can be confirmed by the actual use of poly(MPC-co-BMA) in a human being but also can be evaluated using as an index, an expression level of a GCase gene in a cultured cell of normal keratinocytes originating in a human being. Such cultured cell may be one in which a variation in an expression level of GCase can significantly be distinguished according to the presence thereof when cultured in the presence of a sample for testing. However, it shall not be restricted, and a cultured cell comprising normal keratinocytes originating in a human epidermis and a 3T3 cell (ATCC CRL-1658) originating in a mouse as a feeder layer (supporting cell) can suitably be used. In these cell lines, a 3T3 cell is suitably cultured on a Petri dish and subjected to mitomycin C treatment according to a method described in, for example, Rheinwald et al., Cell 6, p. 331 to 344 (1975). After thus forming a feeder layer, normal human keratinocytes are inoculated on the above layer and cultured at 37°C under, for example, 95 % air-5 % carbon dioxide gas environment until they become confluent.

Any other auxiliaries which are conventionally used in the cosmetic and dermatological fields, for example, diluents, additives and other physiologically active substances can be added to the preparation according to the present invention as long as an adverse effect is not exerted on a GCase activity or the expression of a GCase gene.

The amounts of the active ingredients described above which are added to the above preparation shall not be restricted since the optimum amounts are varied depending on the preparation forms, for example, cream, toilet water, emulsion, lotion and pack, and in the case of, for example, the lotion, it can usually be 0.005 to 20 % by weight, preferably 0.1 to 5 % by weight based on the total weight of the preparation.

Such preparation of the present invention can prevent or repair the troubles of the skin brought about by a reduction in the expression ability of a GCase gene regardless of causes (in particular, drying or aging).

The present invention shall further be explained below with reference to examples, but the present invention shall not be restricted to these examples.

### Expressing test of GCase gene:

### (1) Cultured cell

Used were normal keratinocytes originating in a human foreskin and 3T3 cells (ATCC CRL-1658) originating in a mouse.

### (2) Culture medium for cultured cell

The culture medium is DMEM-Ham' F12 (3 : 1) containing hydrocoltisone, choleraenterotoxin, an epidermal proliferation factor, insulin and 10 % FBS.

### (3) Cell culture and air exposure

Cultured according to a method described in Rheinwald et al., Cell 6, p. 331 to 344 (1975).

The outline shall be shown below. A confluent culture of 3T3 cells was treated with mitomycin C and used as a feeder layer (supporting cell). Human normal keratinocytes of 1 × 10⁵ cells were inoculated on the 3T3 cell treated with mitomycin C and cultured at 37°C under 95 % air-5 % carbon dioxide gas environment until it became confluent. The culture supernatant was removed to thereby carry out air exposure (drying). The respective samples for testing were added five minutes before exposure. One to which these samples were not added was set as a control. The air exposure was carried out for 0 to 6 hours under the condition of 37°C and a humidity of 95 % or 20 %.

### (4) Measurement of GCase activity

The measurement of the enzyme activity was carried out in a citric acid-phosphoric acid buffer solution (pH 5.6) in all cases. An enzyme solution was incubated in an assay buffer solution, and then 4-methylumbelliferyl-β-D-glucopyranoside was added as a substrate to thereby start the reaction. The reaction was stopped by adding a 200 mM carbonate-bicarbonate buffer solution (pH 10.5). The activity was determined by measuring a fluorescent intensity (ex = 360 nm, em = 450 nm) of 4-methylumbelliferone [W. M. Holleran (J. Lipid. Res. 1992, 33 (8) 1201 to 1209)].

### (5) Measurement of GCase gene expression

The respective samples for testing were added to the culture described above, and then mRNA was extracted from a cell exposed to the air for fixed time by an AGPC method. These mRNA's were subjected to RT-PCR in the following manner.

GCase specific primer was prepared based on a base sequence of GCase (refer to Gene, 136 (1-2), 365-368, 1993: Genbank data bases) reported by Imai K. et al. and Nakamura K. et al. In RT-PCR the mRNA was once converted into cDNA with a reverse transcriptase, and it was set as a template to carry out a conventionally known polymerase chain reaction using the primer described above, followed by measuring mRNA of GCase contained in the cell. A PCR product was subjected to electrophoresis with an agarose gel, and after finishing the electrophoresis, the DNA was dyed with an etidium bromide solution. A band of the DNA was determined using NIH image (analytical program of computer). Correction and control were carried out using glyceryl aldehyde-3-phosphate dehydrogenase (G3PDH).

### (6) Results

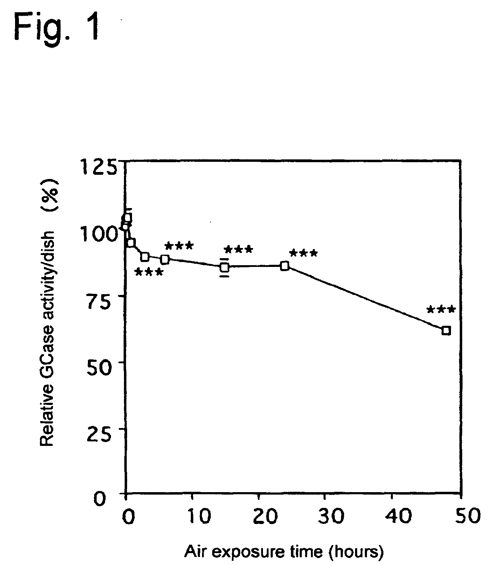

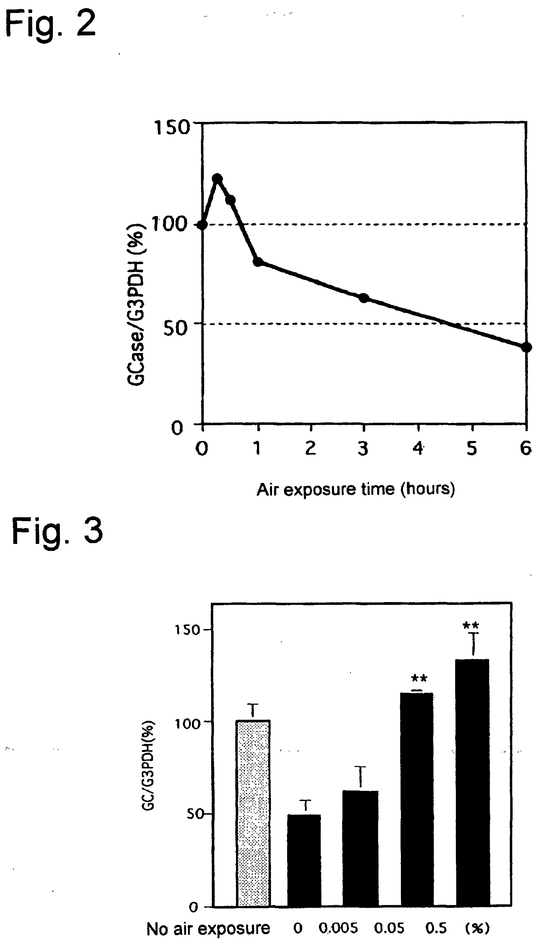

(6-1) Shown in Fig. 1 is a time-dependent change in the GCase activity observed when the cultured cell was exposed to the air (37°C, humidity 95%). It can be found from the drawing that the cultured human keratinocytes exhibit significantly reduced GCase activity upon drying.
(6-2) Shown in Fig. 2 is a time-dependent change in the expression level of the GCase gene in the cultured cell by the air exposure described above. It can be found from the drawing that the expression of the above gene is significantly lowered for a relatively short time.
(6-3) Shown in Fig. 3 is an inhibiting or repairing effect of air exposure (37°C, humidity 95%, 30 minutes) to the cultured cell, which is dependent on the concentration of the. sample poly(MPC-co-BMA) for testing.

It can be found from the drawing that the poly(MPC-co-BMA) not only can enhance dependently on the concentration the expression ability of the GCase gene reduced by exposing the cultured human keratinocytes to the air but also can repair as well the expression ability of the above GCase gene of a normal cell which is not damaged by the air exposure.

The prescription examples of the preparation according to the present invention shall be shown below.

| Prescription Example 1 | |
|---|---|
| 1,3 Butylene glycol | 10.0 % |
| Glycerin | 10.0 % |
| Polyethylene glycol | 5.0 % |
| Cyclomethicone | 5.0 % |
| Cetyl octanoate | 3.0 % |
| Dimethicone | 3.0 % |
| Squalane | 2.0 % |
| Poly(MPC-co-BMA) | 2.0 % |
| Hydrogenated polyisobutene | 0.5 % |
| Polysolvate 60 | 0.3 % |
| Isostearyl isostearate | 0.3 % |
| Behenyl alcohol | 0.5 % |
| Butyl alcohol | 0.3 % |
| Potassium (acrylic acid/alkyl (C10 to C30) acrylate) copolymer | 0.1 % |
| Citric acid | 0.05 % |
| Sodium citrate | 0.05 % |
| Sodium metaphosphate | 0.05 % |
| Edetoate | 0.05 % |
| Sodium hexametaphosphate | |
| (reagent extra grade) | 0.05 % |
| Phenoxyethanol | 0.3 % |
| Methylparaben | 0.1 % |
| Purified water | balance |

| Prescription Example 2 | |
|---|---|
| Sorbitol | 3.0 % |
| Glycerin | 5.0 % |
| Dipropylene glycol | 5.0 % |
| Polyoxyethylene cured castor oil derivative | 0.5 % |
| Ethanol | 10.0 % |
| Poly(MPC-co-BMA) | 0.5 % |
| Perfume | optimum dose |
| Purified water | balance |

| **Prescription Example 3** | |
|---|---|
| Squalane | 10.0 % |
| Isopropyl myristate | 20.0 % |
| Decamethylcyclopentanehexane | 35.0 % |
| Alkyl-modified carboxyvinyl polymer | 0.05 % |
| Sorbitan monooleate | 5.0 % |
| Dipropylene glycol | 3.0 % |
| Poly(MPC-co-BMA) | 1.0 % |
| Purified water | balance |

| Prescription Example 4 | |
|---|---|
| Sedostearyl alcohol | 3.5 % |
| Squalane | 20.0 % |
| Bees wax | 3.0 % |
| Lanolin | 5.0 % |
| Paraben | 0.3 % |
| Polyoxyethylene (20) sorbitan monopalmitate | 2.0 % |
| Stearin monoglyceride | 2.0 % |
| Poly(MPC-co-BMA) | 2.0 % |
| Vitamin A | 0.1 % |
| Perfume | 2.0 % |
| Purified water | balance |

| Prescription Example 5 | |
|---|---|
| Polyvinyl alcohol | 10.0 % |
| Polyethylene glycol | 3.0 % |
| Dipropylene glycol | 5.0 % |
| Ethanol | 10.0 % |
| Poly(MPC-co-BMA) | 4.0 % |
| Arginine | 1.0 % |
| Perfume | optimum dose |
| Purified water | balance |

| Prescription Example 6 | |
|---|---|
| Talc | 3.0 % |
| Titanium dioxide | 5.0 % |
| Red iron oxide | 0.5 % |
| Yellow iron oxide | 1.4 % |
| Black iron oxide | 0.1 % |
| Monostearin polyoxyethylene sorbitan | 0.9 % |
| Triethanolamine | 1.0 % |
| Propylene glycol | 5.0 % |
| Poly(MPC-co-BMA) | 1.0 % |
| Stearic acid | 2.2 % |
| Isohexadecyl alcohol | 7.0 % |
| Glycerin monostearate | 2.0 % |
| Lanolin | 2.0 % |
| Paraffin | 8.0 % |
| Paraben | optimum dose |
| Perfume | optimum dose |

### Industrial Applicability

According to the present invention, provided is a composition or a method which can strengthen the expression of a GCase gene in human epidermal keratinocytes and in its turn can prevent the troubles (for example, a reduction in a skin barrier function) of the skin. Accordingly, the present invention can be used in the cosmetic production industry and the beauty industry.

## Claims

1. A cosmetic or dermatological composition comprising a sufficient amount of a copolymer represented by Formula (I): 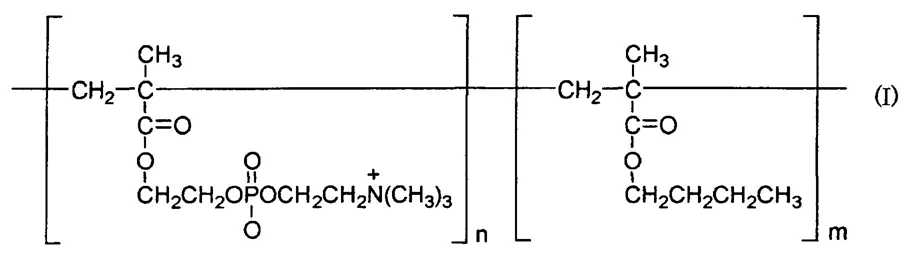 (wherein m and n represent independently integers in which the total thereof allows a number average molecular weight of the above copolymer to be 1,000 to 100,000, and a ratio of m to n falls in a range of 19 : 1 to 1 : 2) for inhibiting or repairing a reduction in the expression of a β-glucocerebrosidase gene, and an auxiliary which is normally used for a cosmetic or a dermatological medical preparation.

2. The composition as described in claim 1, wherein the expression of the β-glucocerebrosidase gene is an event in a human epidermal keratinocyte.

3. A method for inhibiting or repairing a reduction in a skin barrier function or strengthening the skin barrier function, comprising the steps of
applying a composition comprising a copolymer represented by Formula (I): 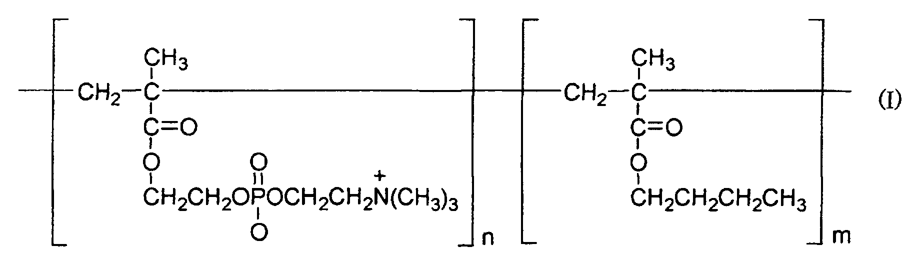 (wherein m and n represent independently integers in which the total thereof allows a number average molecular weight of the above copolymer to be 1,000 to 100,000, and a ratio of m to n falls in a range of 19 : 1 to 1 : 2) on the skin of an individual which is likely to be reduced in the expression ability of a β-glucocerebrosidase gene in epidermal keratinocytes and
strengthening the expression ability of the
β-glucocerebrosidase gene in the epidermal keratinocytes of the skin.

4. The method as described in claim 3, wherein the individual which is likely to be reduced in the expression ability of a β-glucocerebrosidase gene in epidermal keratinocytes is a human being having epidermal keratinocytes damaged by drying or aging.

5. A use of a copolymer represented by Formula (I): 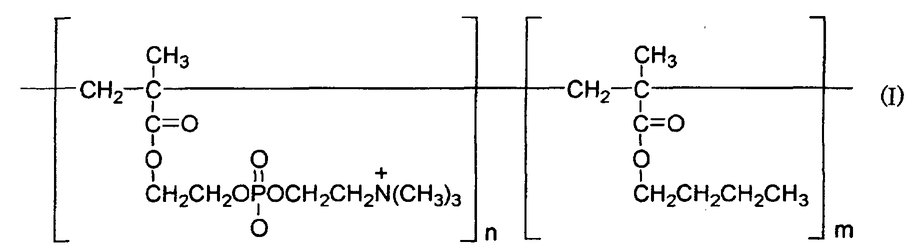 (wherein m and n represent independently integers in which the total thereof allows a number average molecular weight of the above copolymer to be 1,000 to 100,000, and a ratio of m to n falls in a range of 19 1 to 1 : 2) as an active ingredient for preparing a cosmetic or a dermatological medical preparation for inhibiting or repairing a reduction in a skin barrier function or strengthening the skin barrier function.

6. The use as described in claim 5, wherein inhibiting or repairing a reduction in the skin barrier function or strengthening the skin barrier function is achieved by strengthening the expression ability of a β-glucocerebrosidase gene in epidermal keratinocytes.
